# EUROPEAN PATENT APPLICATION

(11) **EP 1 797 909 A2**
(43) Date of publication of application: **20.06.2007**
(21) Application number: 06256045.3
(22) Date of filing: 27.11.2006
(51) Int. Cl.: A61L 27/38, A61L 27/54

(54) **Compositons and methods to create a vascularized environment for cellular transplantation**

(30) Priority: 28.11.2005 US 287797
(71) Applicant: LifeScan, Inc., Milpitas, CA 95035 (US)
(72) Inventor: Xu, Jean, Hillsborough, NJ 08844 (US); Harris, Ian R., Berwyn, PA 19312 (US)
(74) Representative: Tunstall, Christopher Stephen

(57) **Abstract**

The present invention generally relates to biocompatible devices suitable for promoting the movement of cells from a first location to a second location and their retention in the second location. In particular, the present invention relates to an implant, comprising a biocompatible support loaded with at least one pharmaceutical agent capable promoting the movement of cells from a first location to a second location and their retention in the second location. The at least one pharmaceutical agent promotes vascularization at or near the implant site. Vascularization of the implanted support results in enhanced survival of cells optionally incorporated within the support. Methods for treating a disease or injury via implanting the support of the present invention are also provided.

## Description

### FIELD OF THE INVENTION

The present invention generally relates to biocompatible devices suitable for supporting and implanting cells into a mammal wherein the device has incorporated into it at least one pharmaceutical agent. In particular, the pharmaceutical agent is a chemoattractant for cells. The chemoattractant promotes the chemotaxis of cells to, and their retention in the implant site.

### BACKGROUND OF THE INVENTION

There is a need to develop devices for transplanting cells, organoids, organs or tissue to create artificial organs when the patient's own organ function is lost or impaired due to disease or injury. Such devices are able to deliver the transplanted cells to a specific site within the recipient. The therapeutic applications for the transplanted cells can include, for example, hepatocytes for the treatment of liver failure, chromaffin cells for chronic pain, cells that produce clotting factors for hemophilia, islets or insulin producing cells for the treatment of diabetes, cells that produce nerve growth factors for neurodegenerative disease such as Parkinson's or Alzheimer's disease, and cardiovascular cells for the treatment of cardiovascular diseases.

Many have tried to overcome the limitations of cell transplantation by creating a three-dimensional matrix or support, to which cells can attach *in vitro.* However, the physical properties of the support limit diffusion of oxygen and nutrients to the cells, due to lack of sufficient vasculature to carry such nutrients and to remove wastes, resulting in an ischemic environment for the transplanted cells. Consequently, the cells quickly die or cease to function once they are implanted into the recipient.

Previous attempts have focused on incorporation of growth factors into the support. These factors promote an angiogenic response to enhance implant survival. This approach has shown marginal effectiveness only when combinations of growth factors, such as platelet derived growth factor (PDGF-BB) plus vascular endothelial growth factor (VEGF) or basic fibroblast growth factor (bFGF), are delivered from a three-dimensional construct. The main challenge in tissue engineering is that recapitulating the normal angiogenesis response required for new vessel formation is very difficult to achieve because the precise nature, dose, and sequence of growth factors needed for stable vessel formation is poorly defined.

Stem cells are able to bring about the generation of new vascular tissue. The mechanism is unclear, but it is postulated that the stem cells are able to differentiate into endothelial cells and secrete angiogenic factors. This stem cell-mediated neo-vascularization is thought to be a mechanism by which tissues normally repair in response to an injury. Damage to tissues, such as, for example a decrease in tissue oxygen levels, results in an increase in expression of stromal cell-derived factor-1 (SDF-1). This protein is a potent chemoattractant for stem cells and mediates their retention in tissues via binding to the chemokine receptor CXCR4 (Askari et al (2003), Lancet 363: 697-703; Jackson et al (2001), J. Clin Invest. 107: 1395-1402; Quaini et al (2002), N. Engl. J. Med. 346: 5-15). Once normal tissue oxygen levels are restored, the expression of SDF-1 declines to basal levels and the proitein is cleared from the body. Any stem cells present at the site of neo-vascularization are no longer retained once this occurs. Consequently, this mechanism may not besufficient to promote the vascularization of an implant that is introduced into a recipient mammal's body without further intervention.

Results of work described herein indicate that vascularization at or around an implant is enhanced by promoting the chemotaxis of cells to, and their retention in the implant site. The construct of the present invention provides a biodegradable support loaded with desirable agent(s), designed to increase the vasculature surrounding the construct, leading to enhanced function of the construct.

### SUMMARY OF THE INVENTION

The current invention avoids the problems encountered by researchers in the past by using a device to create a vascularized transplant site within a mammal that improves the survivability of therapeutic cells. The current invention provides an implant for the transplantation of cells, tissues, organoids, or organs for the effective treatment of a disease or injury. The implant preferably comprises a porous support, that contains a pharmaceutical agent or agents that are designed to create a vascularized bed around and within the implant. The vascularized bed can significantly enhance the survival of transplanted cells, tissues, organoids or organs that are optionally and preferably contained within the implant. Preferred pharmaceutical agents include factors that are chemoattractants for stem or progenitor cells. Exemplary embodiments employ a class of chemoattractive agents that are ligands for the chemokine receptor CXCR4. The most preferred embodiment employs the CXCR4 ligand stromal cell-derived factor-1 (SDF-1). An amino acid sequence for human SDF-1 is available in the NCBI database at accession number P48061, gi:1352728. See website at ncbi.nlm. nih.gov. This sequence for human SDF- 1 is provided below as SEQ ID NO: 1

SEQ ID1:

1 MNAKVVVVLV LVLTALCLSD GKPVSLSYRC PCRFFESHVA 41
RANVKHLKIL NTPNCALQIV ARLKNNNRQV CIDPKLKWIQ 81
EYLEKALNKR FKM

In an alternate embodiment, the implant of the present invention may be further incorporated with pharmaceutical compounds that reduce inflammation, reduce fibrosis and/or to enhance angiogenesis.

The current invention takes into consideration the unique environment that needs to be established to preserve the ectopic functional viability of transplanted cells or tissue by the establishment of a highly vascularized environment in a site that is easily accessible, preferably by minimally invasive techniques. Further, it allows the vasculature to come into close proximity to the transplanted cells or tissue providing optimal access to the oxygen and nutrients required to maintain functional viability for prolonged periods of time.

The support of the implant of the present invention is constructed out of a foam, or a fibrous mat encapsulated by and disposed within a foam. In an alternate embodiment, the support contains a plurality of interconnecting spaces within the walls of the support. These spaces form a volume into which therapeutic cells may be placed and allow the in-growth of vasculature into the support. The support is preferably biodegradable. Biodegradable polymers readily break down over time *in vivo* and the biodegraded segments do not elicit a chronic foreign body reaction.

The implant of the present invention can be any shape, as long as it is of sufficient size to promote vascularization and incorporate the desired number of therapeutic cells that are optionally incorporated in the implant. The supports of the implants of the present invention can be manufactured by any method known to those of skill in the art, such as, for example, molding, extrusion, weaving and the like.

Where therapeutic cells are desired in the implant, the support may be seeded with cells or tissue prior to transplantation. Alternatively, the support may be transplanted and, subsequent to the transplant, seeded with cells or tissue.

The at least one chemoattractive agent that is incorporated into the implants of the present invention promotes the chemotaxis of autologous cells to the implant site from distant sites within the recipient mammal. "Chemotaxis" is the movement of cells toward a chemical agent in the direction of the highest concentration of the chemical agent. Alternatively and preferably, the cells that move toward the implant site are introduced into the recipient animal. These cells can be allogenic, xenogenic or autologous in origin.

The present invention provides methods to promote vascularization of a site within a mammal using the implants disclosed herein. In a preferred embodiment, at least one pharmaceutical agent that is a chemoattractant for CXCR4 positive cells is incorporated into the support used to construct the implant. Incorporation is achieved by adding an effective amount of the at least one pharmaceutical agent to the raw materials of the support and subsequently fabricating the support. In an alternate embodiment, the support is fabricated and then coated with an effective amount of at least one pharmaceutical agent.

In still another aspect, the present invention provides a method for treating a disease or injury that includes the steps of: implanting in a mammal an implant of the present invention, incorporating at least one pharmaceutical agent that is a chemoattractant for CXCR4 positive cells, optionally incorporating therapeutic cells or tissue and, and administering an effective amount of CXCR4 positive stem cells to the mammal.

In still another aspect, the present invention provides a method for treating a disease or injury that includes the steps of: implanting in a mammal a porous, biocompatible implant of the present invention, incorporating at least one pharmaceutical agent that is a chemoattractant for CXCR4 positive cells and optionally incorporating therapeutic cells or tissue.

### BRIEF DESCRIPTION OF THE FIGURES

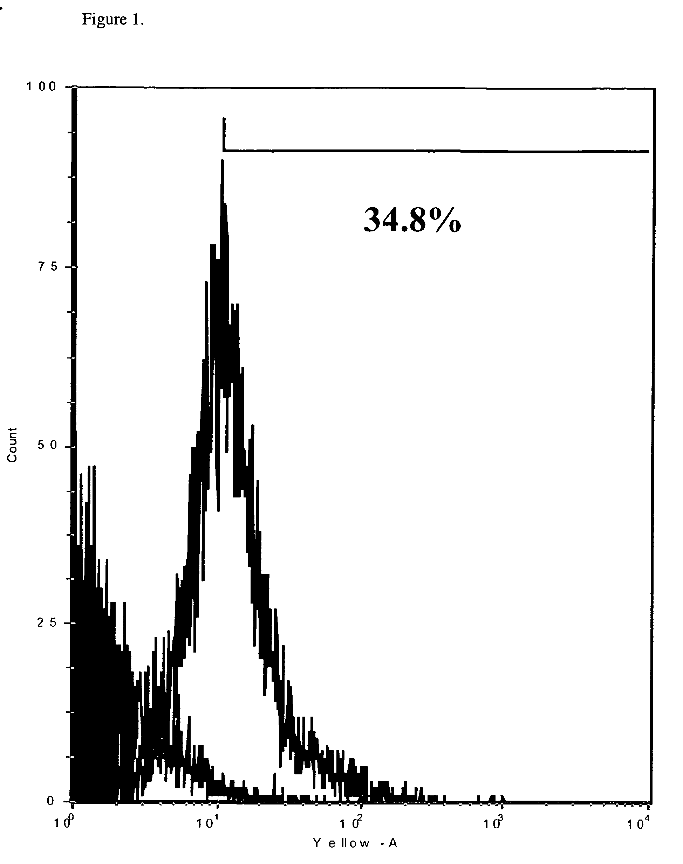

FIGURE 1: ANALYSIS OF CELLULAR CXCR4 BY FLOW CYTOMETRY. The levels of CXCR-4 expression is shown by the black line; the gray line is the corresponding IgG control.

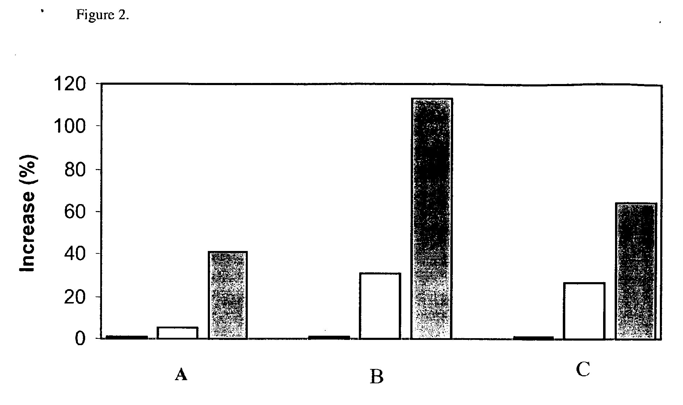

FIGURE 2: SDF-1 MEDIATED CHEMOTAXIS. SDF-1 was added to the wells of a trans-well plate and the migration of human endothelial cells (A), bone marrow derived CXCR4+ cells (B), and bone marrow derived mesenchymal cells (C). The migratory response of the cells toward different dosages of SDF-1 was measured by a trans-well migration assay. Data shown is the percent increase over basal migration in untreated wells (black bars), wells containing 10 ng/ml SDF-1 (white bars) and 100 ng/ml SDF-1 (gray bars).

### DETAILED DESCRIPTION OF THE INVENTION

The present invention is directed to a biocompatible implant, comprising a support and at least one pharmaceutical agent. The at least one pharmaceutical agent in the implant is capable of promoting the chemotaxis of cells to, and their retention in the implant site. Preferably, the implants of the present invention are incorporated with therapeutic cells, and the pharmaceutical agent promotes survival of mammalian cells through its ability to stimulate vascularization at, or around the implant.

In one aspect of the present invention, a biocompatible implant is provided, which is composed of a porous support, loaded with an effective amount of at least one pharmaceutical agent that promotes vascularization of the implant and promotes survival of cells, organoids or organs that are optionally incorporated within the support.

The term "support" as used herein refers to a three-dimensional architecture that is capable of supporting cells on the surface or within the architecture.

The term "porous" as used herein refers to a plurality of inter connecting spaces within the support that enables the distribution of nutrients and cells within the support.

The term "biocompatible" refers to the ability of the support to reside within a mammal so as not to induce toxic or undesirable effects in that mammal.

By "biodegradable" or "absorbable" is meant that the device will be gradually degraded or absorbed by natural biological processes after the device is delivered to a site of interest within a mammal.

The term "implantable" as used herein refers to an implant that is suitable in design, substance and use thereby permitting it to be positioned safely in a mammal. The implant of the present invention can be implanted in a variety of locations in a mammal, particularly where improved, or increased, or new vascularization is desired.

The term "matrix" as used herein refers to the material that comprises the solid component of a support.

The term "therapeutic cell" refers to any cell, organoid or tissue that is employed as an effective treatment of disease or injury.

The term "chemoattractant" refers to a chemical agent that induces movement of cells in the direction of its highest concentration.

In a preferred embodiment, the at least one pharmaceutical agent that is a chemoattractant for CXCR positive cells, which is incorporated into the walls of a support of the present invention. In a most preferred embodiment, the chemoattractant is the protein stromal cell-derived factor-1 (Catalogue no. 350NS010, R&D systems, Minneapolis, MN USA). In analternate embodiment, the chemoattractant is a peptide with the amino acid sequence of the polypeptide SEQ ID1.

### THE SUPPORT

One of ordinary skill in the art will appreciate that the selection of a suitable material for forming the supports of the present invention depends on several factors. The more relevant factors in the selection of the appropriate material include bioabsorption (or biodegradation) kinetics; *in vivo* mechanical performance; cell response to the material in terms of cell attachment, proliferation, migration and differentiation; and biocompatibility. Other relevant factors, which to some extent dictate the *in vitro* and *in vivo* behavior of the material, include the chemical composition, spatial distribution of the constituents, the molecular weight, the degree of crystallinity, and monomer content in the case of polymeric materials. The surface properties of the materials can also be optimized to achieve the desired hydrophilicity. Synthetic and natural materials that are employed in the construction of the supports of the present invention are disclosed in US 5,770,417, US 6,022,743, US 5,567,612, US 5,759,830, US 6,626,950, US 6,534,084, US 6,306,424, US 6,365,149, US 6,599,323, US 6,656,488, and US 6,333,029). Exemplary methods to construct the polymers used in the device of the present invention are disclosed in US patent application US20040062753 A1 and U.S. Pat. No. 4,557,264.

In one embodiment of the present invention, the support is porous and optionally contains at least one pharmaceutical agent. Alternatively the support is composed of biocompatible fibers (the fibrous component), encapsulated by and disposed within a porous, biocompatible, polymeric matrix (the foam component). The support also includes at least one pharmaceutical agent. The porosity of the support may vary depending on the application and the site of implantation. Porosity can be controlled by a variety of means such as the density of the fibers in the nonwoven component, or the concentration or amount of the polymer solution used in forming the foam component. Cells can attach to both the fibers and to the walls of the matrix encapsulating the fibers. The walls of the matrix incorporate one or more pharmaceutical agents that are locally released at the site of implantation in an effective dose to promote the vascularization of the implanted device and to promote the survival of the incorporated cells.

The supports of the present invention can be biodegradable or non-biodegradable. In the case of a non-biodegradable composite support, either or both of the fibers and the matrix encapsulating the fibers can be made from non-biodegradable materials, e.g., non-biodegradable polymers including, but not limited to, polyethylene, polyvinyl alcohol (PVA), polymethylmethacrylte (PMMA), silicone, polyethylene oxide (PEO), polyethylene glycol (PEG), and polyurethanes.

Preferably however, the supports of the present invention are biodegradable. Examples of suitable biodegradable materials include biodegradable synthetic polymers such as aliphatic polyesters, polyalkylene oxalates, polyamides, polycarbonates, polyorthoesters, polyoxaesters, polyamidoesters, polyanhydrides and polyphosphazenes.

Aliphatic polyesters are among the preferred biodegradable polymers for use in making the supports according to the present invention. Aliphatic polyesters can be homopolymers or copolymers (random, block, segmented, tapered blocks, graft, triblock, etc.) having a linear, branched or star structure. Suitable monomers for making aliphatic homopolymers and copolymers include, but are not limited to, lactic acid, lactide (including L-, D-, meso and L, D mixtures), glycolic acid, glycolide, ε-caprolactone, p-dioxanone, trimethylene carbonate, polyoxaesters, δ-valerolactone, β-butyrolactone, ε-decalactone, 2,5-diketomorpholine, pivalolactone, α, α-diethylpropiolactone, ethylene carbonate, ethylene oxalate, 3-methyl-1,4-dioxane-2, 5-dione, 3,3-diethyl-1,4-dioxan-2, 5-dione, γ-butyrolactone, 1,4-dioxepan-2-one, 1,5-dioxepan-2-one, 6,6-dimethyl-dioxepan-2-one and 6,8-dioxabicycloctane-7-one. These polymers can be readily synthesized by those trained in the art or commercially purchased from various suppliers, such as Birmingham Polymers, Inc (Birmingham, Al).

Elastomeric copolymers are also particularly useful in making the supports of the present invention. Elastomeric supports are less abrasive to the site of implantation as compared to stiff non-elastomeric supports and can be more readily handled at the surgical site. Such polymers are taught in US patent No 6,365,149, which is hereby incorporated by reference. This is particularly advantageous when placing a support in a site such as subcutaneous pouch, omentum, or wrapping the support around an organ such as the pancreas. Suitable elastomeric polymers include those with an inherent viscosity in the range of about 1.2 dL/g to about 4 dL/g, more preferably about 1.2 dL/g to about 2 dL/g and most preferably about 1.4 dL/g to about 2 dL/g, as determined at 25°C in a 0.1 gram per deciliter (g/dL) solution of polymer in hexafluoroisopropanol (HFIP). Further, suitable elastomers exhibit a high percent of elongation and a low modulus, while possessing good tensile strength and good recovery characteristics.

In a preferred embodiment where elastomeric copolymers are used, the elastomer from which the support is formed preferably exhibits a percent elongation greater than about 200 percent, and more preferably greater than about 500 percent. In addition to these elongation and modulus properties, suitable elastomers also should have a tensile strength greater than about 500 psi., preferably greater than about 1,000 psi.; and a tear strength of greater than about 50 lbs/inch, preferably greater than about 80 lbs/inch.

Exemplary biodegradable, biocompatible elastomers include, but are not limited to, elastomeric copolymers of ε-caprolactone and glycolide with a mole ratio of ε-caprolactone to glycolide of from about 35/65 to about 65/35, more preferably from 35/65 to 45/55; elastomeric copolymers of ε-caprolactone and lactide where the mole ratio of ε-caprolactone to lactide is from about 35/65 to about 65/35 and more preferably from 35/65 to 45/55; elastomeric copolymers of lactide and glycolide where the mole ratio of lactide to glycolide is from about 95/5 to about 85/15; elastomeric copolymers of p-dioxanone and lactide where the mole ratio of p-dioxanone to lactide is from about 40/60 to about 60/40; elastomeric copolymers of ε-caprolactone and p-dioxanone where the mole ratio of s-caprolactone to p-dioxanone is from about from 30/70 to about 70/30; elastomeric copolymers of p-dioxanone and trimethylene carbonate where the mole ratio of p-dioxanone to trimethylene carbonate is from about 30/70 to about 70/30; elastomeric copolymers of trimethylene carbonate and glycolide where the mole ratio of trimethylene carbonate to glycolide is from about 30/70 to about 70/30; elastomeric copolymers of trimethylene carbonate and lactide where the mole ratio of trimethylene carbonate to lactide is from about 30/70 to about 70/30, or blends thereof. Exemplary elastomeric polymers and methods to form supports from elastomeric polymers are disclosed in US 6,534,084; US 6,365,149; US6 423,252 and US 6,355,699.

In another embodiment, it is desirable to use polymer blends to form structures which transition from one composition to another composition in a gradient-like architecture. Composite supports having this gradient-like architecture are particularly advantageous in tissue engineering applications to repair or regenerate the structure of naturally occurring tissue. For example, by blending an elastomeric copolymer of ε-caprolactone and glycolide with an elastic copolymer of ε-caprolactone and lactide (e.g., glycolide with an elastic copolymer of ε-caprolactone and lactide (e.g., with a mole ratio of about 5/95) a support may be formed that transitions from a softer spongy material to a stiffer more rigid material. Clearly, one of ordinary skill in the art having the benefit of this disclosure will appreciate that other polymer blends may be used for similar gradient effects, or to provide different gradients, e.g. different degradation profiles, stress response profiles or different degrees of elasticity. Such structures have been disclosed in WO02051463, which is hereby incorporated by reference.

With a composite support, the fibers encapsulated by a porous matrix can be organized in a form selected from threads, yarns, nets, laces, felts and nonwoven mats. Preferably, the fibers are in the form of a nonwoven fibrous mat. Known wetlay or dry-lay fabrication techniques can be used to prepare the fibrous nonwoven mat of the composite support of the present invention ("Non-woven textiles", by Radko Krcma, Textile Trade Press, Manchester, UK, 1967).

Preferably, the fibers that form the nonwoven fibrous mat of a composite support are made of biodegradable polymers such as polylactic acid (PLA), polyglycolic acid (PGA), ε-polycaprolactone (PCL), polydioxanone (PDO), or copolymers and blends thereof.

The porous matrix of a support as described herein is preferably in the form of a polymeric foam. A foam support or the foam matrix of a composite support can be formed by a variety of techniques well known to those having ordinary skill in the art. For example, the polymeric starting materials may be formed by lyophilization, supercritical solvent foaming, gas injection extrusion, gas injection molding or casting with an extractable material (e.g., salts, sugar or similar suitable materials).

In one embodiment, the support of the present invention is made by a polymer-solvent phase separation technique, such as lyophilization. The steps involved in the preparation of these foams include choosing the appropriate solvents for polymer lyophilization and preparing a homogeneous solution of the polymer in the solvent. The polymer solution is then subjected to a freezing and vacuum-drying cycle. The freezing step phase-separates the polymer solution and the vacuum-drying step removes the solvent by sublimation and/or drying, thus leaving a porous, polymer matrix, or an interconnected, open-cell, porous foam.

In one embodiment, the composite support of the present invention is made using a polymer-solvent phase separation technique, such as lyophilization. The steps involved in the preparation of these foams include choosing the appropriate solvents for polymer lyophilization and preparing a homogeneous solution of the polymer in the solvent. The polymer solution is then subjected to a freezing and vacuum-drying cycle. The freezing step phase-separates the polymer solution and the vacuum-drying step removes the solvent by sublimation and/or drying, thus leaving a porous, polymer matrix, or an interconnected, open-cell, porous foam.

Suitable solvents dissolve the biodegradable polymer for forming the foam matrix, and maintain the fibers (e.g., of a nonwoven mat) of the composite support.

Exemplary solvents to be matched with the appropriate polymer include, but are not limited to, hexafluoroisopropanol (HFIP), cyclic ethers (e.g., tetrahydrofuran (THF) and dimethylene fluoride (DMF)), acetone, methylethyl ketone (MEK), 1,4-dioxane, dimethlycarbonate, benzene, toluene, N-methyl pyrrolidone, dimethylformamide, chloroform, and mixtures thereof. One of ordinary skill in the art could make the appropriate pairings. Among these solvents, a preferred solvent is 1,4-dioxane. A homogeneous solution of the polymer in the solvent is prepared using standard techniques.

The applicable polymer concentration or amount of solvent to be used may vary, depending on the polymer and the solvent used. In general, the amount of polymer in the solution is in the range of about 0.01 % to about 90% by weight and, preferably, is in the range of about 0.1 % to about 30% by weight, depending on factors such as the solubility of the polymer in a given solvent and the final properties desired in the foam support including the release profile of the added pharmaceutical agent.

In yet another embodiment of the invention, the foam component or the fibrous component of a support can be chemically cross-linked or combined with hydrogels, such as polyethylene glycol and poly (N-isopropylacryalmide)

In yet another embodiment, the support of the current invention can be combined with a biopolymer selected from the group consisting of hyaluronic acid, collagen, recombinant collagen, cellulose, elastin, alginates, chondroitin sulfate, chitosan, and small intestine submucosa (SIS).

The supports of the present invention preferably include interconnecting pores or voids, which facilitate the incorporation of cells into the support, as well as the transport of nutrients and/or expansion of cells within the support. The interconnected pores preferably range in size from about 50 to 1000 microns, preferably 50 to 400 microns, and preferably constitute about 70 to 95 percent of the total volume of the support. The range of the pore size in the support can be manipulated by modifying process steps during the preparation of the support.

Support materials have been extensively studied as tissue templates, conduits, barriers, and reservoirs useful for tissue repair. In particular, synthetic and natural materials in the form of foams, sponges, gels, hydrogels, textiles, and nonwoven structures have been used *in vitro* and *in vivo* to reconstruct or regenerate biological tissue, as well as to deliver chemotactic agents for inducing tissue growth, such as the methods disclosed in US 5,770,417, US 6,022,743, US 5,567,612, US 5,759,830, US 6,626,950, US 6,534,084, US 6,306,424, US 6,365,149, US 6,599,323, US 6,656,488, and US 6,333,029). Exemplary polymers used in the device of the present invention are disclosed in US patent application US20040062753 A1 and U.S. Pat. No. 4,557,264.

To form a support incorporated with a pharmaceutical agent, the pharmaceutical agent can be mixed with the polymer solution prior to forming the support. Alternatively, a pharmaceutical agent could be coated onto a fabricated support, preferably in the presence of a pharmaceutical carrier. The pharmaceutical agent may be present as a liquid, a finely divided solid, or any other appropriate physical form. Alternatively, excipients may be added to the support to alter the release rate of the pharmaceutical agent. In an alternate embodiment, the support incorporated with at least one pharmaceutical agent that is a chemoattractant for CXCR4 positive cells is further incorporated with at least one pharmaceutical compound that is an anti-inflammatory compound, such as, for example compounds disclosed in US 6,509,369.

In a further embodiment, the support incorporated with at least one pharmaceutical agent that is a chemoattractant for CXCR4 positive cells is further incorporated with at least one pharmaceutical compound that is an anti-apoptotic compound, such as, for example compounds disclosed in US 6,793,945.

In a further embodiment, the support incorporated with at least one pharmaceutical agent that is a chemoattractant for CXCR4 positive cells is further incorporated with at least one pharmaceutical compound that is an inhibitor of fibrosis, such as, for example compounds disclosed in US 6,331,298.

In a further embodiment, the support incorporated with at least one pharmaceutical agent that is a chemoattractant for CXCR4 positive cells is further incorporated with at least one pharmaceutical compound that is capable of enhancing angiogenesis, such as, for example compounds disclosed in US20040220393 and US20040209901.

In a further embodiment, the support incorporated with at least one pharmaceutical agent that is a chemoattractant for CXCR4 positive cells is further incorporated with at least one pharmaceutical compound that is an immunosuppressive compound, such as, for example compounds disclosed in US20040171623.

In a further embodiment, the support incorporated with at least one pharmaceutical agent that is a chemoattractant for CXCR4 positive cells is further incorporated with at least one pharmaceutical compound that is a growth factor, such as, for example members of the TGF-β family, including TGF-β1, 2, and 3, bone morphogenic proteins (BMP-2, -3, -4, -5, -6, -7, -11, -12, and -13), fibroblast growth factors-1 and -2, platelet-derived growth factor-AA, and -BB, platelet rich plasma, insulin growth factor (IGF-I, II) growth differentiation factor (GDF-5, -6, -8, -10, -15) vascular endothelial cell-derived growth factor (VEGF), pleiotrophin, endothelin. Other pharmaceutical compounds can include, for example, nicotinamide, hypoxia inducible factor 1-alpha, glucagon like peptide-I (GLP-1) and II, Exendin-4, retinoic acid, parathyroid hormone, tenascin-C, tropoelastin, thrombin-derived peptides, cathelicidins, defensins, laminin, biological peptides containing cell- and heparin-binding domains of adhesive extracellular matrix proteins such as fibronectin and vitronectin, MAPK inhibitors, such as, for example, compounds disclosed in US20040209901 and US20040132729.

In a preferred embodiment, the at least one pharmaceutical agent is a chemoattractant for cells, which is incorporated into the walls of a support of the present invention. In a most preferred embodiment, the chemoattractant is the protein stromal cell-derived factor-1 (Catalogue no. 350NS010, R&D systems, Minneapolis, MN USA).

The amount of the chemoattractant effective to promote cell chemotaxis may vary, depending on the support used and the nature of the inhibitor, and can be readily determined by one skilled in the art. Generally speaking, an effective amount of a cemoattractant is about 1 pico gram/cm² to about 1 mg/cm² of the support, and more typically about 1 pico gram/cm² to about 10 µg/cm² of the support.

The biodegradable supports of the present invention can undergo gradual degradation (mainly through hydrolysis) with concomitant release of the dispersed pharmaceutical agent for a sustained or extended period, which is sufficient to promote vascularization of the implant or the area around the implant. Preferably the implant promotes prolonged delivery of the at least one pharmaceutical agent, e.g. over 1 to 5,000 hours, preferably 2 to 800 hours, of effective amounts, e.g. 0.0001 mg/kg/hour to 10 mg/kg/hour, of the pharmaceutical agent. This dosage form can be administered as is necessary depending on the subject being treated, the severity of the affliction, the judgment of the prescribing physician, and the like. Following this or similar procedures, those skilled in the art will be able to prepare a variety of formulations.

### THERAPEUTIC USE OF THE IMPLANTS

The present invention provides an implant, comprising a support that has incorporated into it at least one pharmaceutical agent that is capable of promoting vascularization. The implant of the present invention promotes vascularization by promoting the chemotaxis of stem or progenitor cells to, and their retention in the implant site. In one embodiment of the invention, the stem cells are CXCR4 positive bone marrow derived stem cells from sites within the recipient animal, such as those disclosed in Askari et al (2003), Lancet 363: 697-703. In an alternate embodiment, the stem or progenitor cells are introduced into the mammal during implantation. However, the stem or progenitor cells can be introduced or any time after implantation of the support. These stem or progenitor cells can be autologous, allogenic or xenogenic in origin. The amount and type of cell administered will vary depending on the subject being treated, the severity of the affliction, the judgment of the prescribing physician, and the like. Those skilled in the art will be able to prepare a variety of formulations.

In a further embodiment the recipient is treated with factors to increase the proliferation of a sub-type of stem cells, such as, for example, mesenchymal stem cells, as disclosed in US patent 6,261,549. The cells can be purified from the patient prior to the preparation of the implant of the present invention. These cells are then administered together with the implant.

The support of the present invention can be implanted in an animal. Alternatively and preferably, the implant contains therapeutic cells or tissues that are incorporated into the support prior to, or at the time of, implantation. The cells may be cultured under standard conditions known to those skilled in the art in order to increase the number of cells or induce differentiation to the desired phenotype prior to seeding into the support. Alternatively, the therapeutic cells can be injected directly into the support and then cultured *in vitro* under conditions that promote proliferation and deposition of the appropriate biological matrix prior to implantation. One skilled in the art can readily recognize such conditions.

Alternatively, after preparation of the implant of the present invention, the implant without therapeutic cells can be implanted at a site within a patient. The implant can be maintained in the patient until it becomes infiltrated with vascular tissue. At this time, therapeutic cells can be introduced into the implant by appropriate means, such as, for example, by injection.

To introduce the therapeutic cells into a support, the support is contacted and incubated with a suspension containing the cells, or clusters of cells. The incubation can be performed for a short period of time (< 1 day) just prior to implantation, or for longer a period (> 1 day) to allow for enhanced cell attachment, cell proliferation and extracellular matrix synthesis within the seeded support prior to implantation.

The present invention provides a method for treating a disease or injury by inserting at a site in the patient, an implant of the present invention, optionally with a therapeutically effective amount of stem or progenitor cells. Alternatively and preferably, the implant is seeded with therapeutic cells, tissues or organs. In another alternate embodiment, the implant without therapeutic cells is implanted and maintained in the patient until it becomes infiltrated with vascular tissue. At this time, cells are introduced into the implant by appropriate means, such as, for example, by injection. The site where the device can be implanted can be any clinically relevant site, such as, for example, the liver, the natural pancreas, the renal subcapsular space, the mesentery, the omentum, a subcutaneous pocket, or the peritoneum. The site can be an immunologically privileged site, either naturally existing or created using, for example, Sertoli cells.

The therapeutic cells useful for administration in this invention include autologous, allogeneic, or xenogeneic cells. In the case that the invention is intended to treat diabetes, the cells can be stem cells, pancreatic precursor/progenitor cells, genetically engineered insulin producing cells, primary or expanded partially or fully differentiated islets or insulin producing cells.

Such treatment may also be used for other types of cell therapy, including, for example, hepatocytes for the treatment of liver failure, chromaffin cells for chronic pain, cells that produce clotting factors for hemophilia, and cells that produce nerve growth factors for neurodegenerative disease such as Parkinson's or Alzheimer's disease, as well as fibroblasts, myofibroblasts, cardiovascular cells, neural cells, and neural precursor cells.

Other cells that can be therapeutically effective for different applications include, but are not limited to, progenitor cells, precursor cells, stem cells, bone marrow cells, umbilical cord blood cells, angioblasts, endothelial cells, osteoblasts, smooth muscle cells, kidney cells, fibroblasts, myofibroblasts, cardiovascular cells, neural cells, neural precursor cells, amniotic cells and post-partum placental cells. In a further embodiment of the present invention, the cells may be genetically engineered to produce a therapeutic protein, or to down-regulate the recipient's immune response.

The cells introduced into the implant of the present invention need not be limited to just one cell type. The implants of the present invention enable the construction of "artificial organs", wherein the cell types found in normal organs, such as, for example, the liver, the pancreas, the kidney, are incorporated into the support of the implant. In an alternative embodiment, cells that are capable of modulating the recipient's immune response, such as, for example, mesenchymal stem cells and suppressor T cells, as disclosed in US 6,328,960, US 6,281,012 and US 6,685,936.

### EXAMPLES

The present invention is further illustrated but not limited by the following examples.

In the examples, the polymers and monomers were characterized in chemical composition and purity (NMR, FTIR), thermal analysis (DSC) and molecular weight by conventional analytical techniques.

Inherent viscosities (I.V., dL/g) of the polymers and copolymers were measured using a 50 bore Cannon-Ubbelhode dilution viscometer (Labovisco, Zoetermeer, Netherlands) immersed in a thermostatically controlled water bath at 30°C utilizing chloroform or hexafluoroisopropanol (HFIP) as the solvent at a concentration of 0.1 g/dL.

In these examples certain abbreviations are used. These include PCL to indicate polymerized ε-caprolactone and PGA to indicate polymerized glycolide. Additionally, the ratios in front of the copolymer identification indicate the respective mole percentages of each constituent.

### EXAMPLE 1: FACS ANALYSIS OF CXCR4+ CELLS.

Fluorescence-activate cell sorting detection of CXCR4+ was performed after 10 days in culture. A total of 2 to 3x10⁵ cells were resuspended with 200 µl of Dulbecco's PBS (Invitrogen, Rockville, MD) containing 10% FBS and 0.01 % NaN₃ and incubated for 20 minutes at 4°C with phycoerythrin- conjugated monoclonal antibody against CXCR4 (BD Biosciences, San Diego, CA). After staining, the cells were fixed in 2% paraformaldehyde. Quantitative FACS was performed on a FACStar flow cytometer (BD Biosciences, San Diego, CA). All groups were studied in triplicate. Results are shown as fluorescence histograms (black, CXCR-4 expression; red, respective 1gG control). *Ex vivo* expanded cells were 34.8% positive for CXCR4 (Figure 1).

### EXAMPLE 2: CHEMOATTRACTIVE ANALYSIS.

Migration was studied using a modified 48 -trans-well assay. SDF-1 was diluted to appropriate concentrations in Endothelial Basal Medium-2 (Cambrex, Walkersville,

MD) supplemented with 0.1% BSA, and 700 µl of the final dilutions were placed in the lower wells. CXCR4+ cells were stained by dioctadecyl-tetramethylindo-carbocyanine perchlorate (DiI) (Molecular Probes, Inc. Eugene, OR) and 5x10⁴ cells were suspended in 300 µl of EBM-2 supplemented with 0.1 % BSA and reseeded onto HTS FluoroBlok insert (3.0 µm pore, BD Biosciences, San Diego, CA). After incubation for 5 hours at 37 °C, the filter was removed and the number of cells adhering to the undersurface of the filter, corresponding to cells that have migrated was measured by flow cytometry at 527 nm. SDF-1 induced the migration of human endothelial cells (A), bone marrow derived CXCR4+ cells (B), and bone marrow derived mesenchymal cells (C). The migratory response of the cells toward different dosages of SDF-1 was measured by a transwell migration assay. *Ex vivo* expanded CXCR4+ cells demonstrated a potent dose-dependent migration toward SDF-1 compared to endothelial cells or mesenchymal cells (Figure 2).

### EXAMPLE 3: FABRICATION A SUPPORT CONTAINING SDF-1.

The polymer that is used to manufacture the foam component is a 35/65 PCL/PGA copolymer produced by Birmingham Polymers Inc. (Birmingham, AL), with an I.V. of 1.45 dL/g. A 5/95 weight ratio of 35/65 PCL/PGA in 1,4-dioxane solvent is weighed out. The polymer and solvent are placed into a flask, which in turn is put into a water bath and stirred for 5 hours at 70 °C to form a solution. The solution is then filtered using an extraction thimble (extra coarse porosity, type ASTM 170-220 (EC)) and stored in a flask. Next, SDF-1 is added in the range of 100 ng to 10 µg/ml to the polymer solution.

As an alternative, the SDF-1 is coated onto the support by dipping the supports in the solution containing SDF-1 at the desired concentration.

A laboratory scale lyophilizer (Model Duradry, FTS Kinetics, Stone Ridge, NY) is used to form the foam support. The polymer solution is added into a 4-inch by 4-inch aluminum mold to a height of 2 mm and subjected to the following freeze-dry protocol: 1) -17°C for 60 minutes, 2) -5°C for 60 minutes under vacuum 100 mT, 3) 5 °C for 60 minutes under vacuum 20 mT, 4) 20 °C for 60 minutes under vacuum 20 mT.

After the cycle is complete, the mold assembly is taken out of the freeze dryer and allowed to de-gas in a vacuum hood for 2 to 3 hours. The foam support is stored under nitrogen. The pore size of this composite support is determined using Mercury Porosimetry analysis. The range of pore size should be 1-300 µm with a median pore size of 45 µm. The thickness of the support should be approximately 1.5 µm.

### EXAMPLE 4: ISLET ISOLATIONS.

Murine islets are isolated from Balb/c By/J (Balb) mice according to an improved method for isolation of mouse pancreatic islets (Transplantation 40: 437-438, 1985). Briefly, 3 ml of collagenase type V (Sigma-Aldrich, St. Louis, MO) solution at 1 mg/ml is slowly introduced into the common bile duct after occlusion of the distal end just proximal to the duodenum. The distended pancreas is excised and the digestion performed in a water-bath at 37°C for 10-15 min. Islet purification is achieved using a two-step, discontinuous density gradient of Ficoll (Sigma-Aldrich, St. Louis, MO). Islets are colleted from the interface between 1.096/1.037 g/ml layer. To ensure 100% purity of the preparation, islets are hand picked and counted under an inverted microscope (Nikon TE200-S). The islets are cultured overnight in CMRL medium with 10% fetal bovine serum, penicillin, streptomycin, L-glutamine, and 25 mM HEPES buffer (Invitrogen, Rockville, MD). Typical yields are between 180 to 270 islets per pancreas. Islets are cultured in CMRL media (Invitrogen, Rockville, MD) containing 10% FBS. One day before transplantation, islets are treated with 200 ng/ml recombinant human SDF-1 (R&D Systems, Minneapolis, MN).

### EXAMPLE 5: ISOLATION AND PURIFICATION OF CXCR4 POSITIVE CELLS.

Human CXCR4+ cells are obtained from human bone marrow (purchased from Cambrex, Walkersville, MD), using the following method. Total bone marrow mononuclear cells are purified from human bone marrow by density-gradient centrifugation with Histopaque-1077 (Sigma-Aldrich, St. Louis, MO) and plated on culture dishes coated with human fibronectin (Invitrogen, Rockville, MD). The cells are cultured under hypoxic conditions (3% O₂, 95% N₂, and 5% CO₂). for seven days in Endothelial Basal Medium-2 (EBM-2, Cambrex, Walkersville, MD) supplemented with 5% FBS, human vascular endothelial growth factor (VEGF-A), human fibroblast growth factor-2, human epidermal growth factor, insulin-like growth factor-1 (R&D Systems, Minneapolis, MN), ascorbic acid, and antibiotics (Invitrogen, Rockville, MD). Non-adherent cells are removed by changing the culture medium four days after the initial plating. Cells are removed by washing with PBS and new medium applied, and the culture is maintained through day seven.

### EXAMPLE 6: DETECTION OF APOPTOTIC CELL DEATH.

Recombinant interleukin-1β (IL-1β; 50 U/ml), recombinant interferon-γ (IFN-γ; 1x10³ U/ml), and recombinant tumor necrosis factor-α (TNF-α; 10³ u/ml), are purchased from R&D Systems Inc. (Minneapolis, MN) and can be of either human or murine origin. The proteins will be used in combination. In order to test the anti-apoptotic effect of SDF-1 on cells, islets or PANC-1 cells (ATCC, Manassas, VA) will be pretreated with SDF-1 for 24 hours before exposing these cells to cytokine cocktails for another 48 hr prior to harvesting. Control islets will remain untreated for the corresponding periods.

A sample of 300 islet equivalents (IEQ) murine islets or 8x10⁴ PANC-1 cells is plated in 2 ml of culture medium for the assay. Samples will be taken after three days in culture for analysis by ELISA (Roche Diagnostics, Indianapolis, IN.) The ELISA is based on a sandwich-enzyme immunoassay principle, and detects histone-associated DNA fragments in the cell cytoplasm, which are characteristic of the apoptotic process. The results will be expressed as absorbance at 405 nm.

### EXAMPLE 7: SDF-1-MEDIATED MIGRATION OF CXCR4 POSITIVE CELLS TO THE IMPLANT OF THE PRESENT INVENTION IN VIVO.

800 murine islets will be seeded onto 5-mm supports containing SDF-1 for 24 hours before the transplantation. The supports will be transplanted into the fat pads of NOD/SCID mice. After surgery, the mice will receive an intra venous injection of 5x10⁵ bone marrow-derived CXCR4 positive cells labeled with dioctadecyl-tetramethylindo-carbocyanine perchlorate (Molecular Probes, Eugene, OR). Mice will be scarified on days 2 or 14 after surgery and the number of DI-labeled cells will be measured by flow cytometry or PCR analysis.

Publications cited throughout this document are hereby incorporated by reference in their entirety. Although the various aspects of the invention have been illustrated above by reference to examples and preferred embodiments, it will be appreciated that the scope of the invention is defined not by the foregoing description, but by the following claims properly construed under principles of patent law.

## Claims

1. A biocompatible implant, comprising a support and least one pharmaceutical agent that is a chemoattractant.

2. The implant of claim 1, wherein the support is biodegradable.

3. The implant of claim 1 or 2, wherein the support is selected from the group consisting of a foam, or a fibrous mat encapsulated by and disposed within a foam.

4. The implant of claim 2, wherein the support is made of one or more biodegradable polymers.

5. The implant of claim 4, wherein the polymers are selected from the group consisting of hyaluronic acid, collagen, recombinant collagen, cellulose, elastin, alginates, chondroitin sulfate, chitosan, chitin, keratin, silk, small intestine submucosa (SIS), or combinations thereof.

6. The implant of claim 2, wherein the support is made from one or more synthetic polymers.

7. The implant of claim 6, wherein the polymers are selected from the group consisting of aliphatic polyesters, polyalkylene oxalates, polyamides, polycarbonates, polyorthoesters, polyoxaesters, polyamidoesters, polyanhydrides, and polyphosphazenes.

8. The implant of claim 7, wherein the polymers are aliphatic polyesters which are homopolymers or copolymers of monomers selected from the group consisting of lactic acid, lactide, glycolic acid, glycolide, ε-caprolactone, p-dioxanone, trimethylene carbonate, polyoxaesters, δ-valerolactone, β-butyrolactone, ε-decalactone, 2,5,-diketomorpholine, pivalolactone, α,α-diethylpropiolactone, ethylene carbonate, ethylene oxalate, 3-methyl-1,4-dioxane-2,5-dione, 3,3-diethyl-1,4-diozan-2,5-dione, γ-butyrolactone, 1,4-dioxepan-2-one, 1,5-dioxepan-2-one, 6,6-dimethyl-dioxepan-2-one and 6,8-dioxabicylcloctane-7-one.

9. The implant of claim 6, wherein the polymers are elastomers.

10. The implant of claim 9, wherein the elastomers are selected from the group of copolymers consisting of ε-caprolactone and glycolide, ε-caprolactone and lactide, lactide and glycolide, p-dioxanone and lactide, ε-caprolactone and p-dioxanone, p-dioxanone and trimethylene carbonate, trimethylene carbonate and glycolide, trimethylene carbonate and lactide, or combinations thereof.

11. The implant of claim 6, wherein the material comprising the support has a gradient structure, **characterized by** a continuous transition from a first biodegradable polymer composition to a second biodegradable polymer composition.

12. The implant of claim 3, wherein the fibrous mat of the support is made by a wet-lay or dry-lay procedure.

13. The implant of claim 3, wherein the foam of the support is formed by a polymer-solvent phase separation technique, supercritical solvent foaming, gas injection extrusion, gas injection molding, or casting with an extractable material.

14. The implant of any preceding claim, wherein the at least one pharmaceutical agent that is a chemoattractant is a chemokine.

15. The implant of claim 14, wherein the at least one agent is capable of binding to the CXCR4 receptor.

16. The implant of claim 14, wherein the chemokine is stromal cell-derived factor-1.

17. The implant of claim 14, wherein the chemokine is a polypeptide with an amino acid sequence consisting essentially of the amino acid sequence of SDF-1.

18. The implant of any preceding claim, wherein the at least one pharmaceutical agent that is a chemoattractant is incorporated into the support prior to the formation of the support by adding the at least one pharmaceutical agent into a polymer solution for forming the support.

19. The implant of any preceding claim, wherein the at least one pharmaceutical agent that is a chemoattractant is incorporated into the support after the formation of the support by coating the support with the at least one pharmaceutical agent.

20. The implant of any preceding claim, further comprising therapeutic tissue attached to, or incorporated within the support.

21. The implant of claim 20, wherein the therapeutic tissue is cells, tissue isolated from an organ, or isolated organs.

22. A method of making a biocompatible implant capable of promoting the chemotaxis of cells, comprising the steps of:
a. fabricating a support, and
b. incorporating at least one pharmaceutical agent that is a chemoattractant.

23. The method of claim 22, wherein the at least one agent is a chemokine.

24. The method of claim 22, wherein the at least one agent is capable of binding to the CXCR4 receptor.

25. The method of claim 23, wherein the chemokine is a stromal cell-derived factor-1.

26. The method of claim 23, wherein the chemokine is a polypeptide with an amino acid sequence consisting essentiakky of the amino acid sequence of SDF-1.

27. The method of any of claims 22 to 25, wherein support is further treated with at least one pharmaceutical compound that is selected from the group consisting of a growth factor, an anti-rejection agent, an analgesic, an anti-oxidant, an anti-apoptopic agent, an anti-inflammatory agent, and an immunosuppressive drug.

28. A method of making a biocompatible implant capable of promoting the chemotaxis of cells, comprising the steps of:
a. incorporating at least one pharmaceutical agent that is a chemoattractant, and
b. isolating and preparing therapeutic tissue for introduction to the support, and
c. introducing the therapeutic tissue into the support.

29. The method of claim 28, wherein the at least one agent is a chemokine.

30. The method of claim 28, wherein the at least one agent is capable of binding to the CXCR4 receptor.

31. The method of claim 29, wherein the chemokine is stromal cell-derived factor-1.

32. The method of claim 29, wherein the chemokine is a polypeptide with an amino acid sequence consisting essentially of the amino acid sequence of SDF-1.

33. The method of any of claims 28 to 32, wherein the support is further treated with at least one pharmaceutical compound that is selected from the group consisting of a growth factor, an anti-rejection agent, an analagesic, an anti-oxidant, an anti-apoptotic agent, an anti-inflammatory agent, and an immunosuppresive drug.
